# EUROPÄISCHE PATENTSCHRIFT

(11) **EP 1 128 841 B1**
(45) Veröffentlichungstag und Bekanntmachung des Hinweises auf die Patenterteilung: **28.04.2004**
(21) Anmeldenummer: 99955585.7
(22) Anmeldetag: 10.11.1999
(51) Int. Cl.: A61K 38/37, A61K 38/36, A61K 38/48, A61P 7/04, A61K 38/57

(54) **PHARMAZEUTISCHE PRÄPARATION, ENTHALTEND EINEN REZEPTOR-ANTAGONISTEN ZUR BEHANDLUNG VON BLUTGERINNUNGSSTÖRUNGEN**
PHARMACEUTICAL PREPARATION CONTAINING A RECEPTOR-ANTAGONIST FOR TREATING BLOOD-CLOTTING DISORDERS
PREPARATION PHARMACEUTIQUE CONTENANT UN ANTAGONISTE DE RECEPTEUR POUR LE TRAITEMENT DE TROUBLES DE LA COAGULATION SANGUINE

(30) Priorität: 10.11.1998 AT 187398
(43) Veröffentlichungstag der Anmeldung: 05.09.2001
(73) Patentinhaber: Baxter Aktiengesellschaft, 1221 Wien (AT)
(72) Erfinder: SCHWARZ, Hans-Peter, A-1180 Wien (AT); TURECEK, Peter, A-3400 Klosterneuburg (AT); BINDER, Bernd, A-1010 Wien (AT)
(74) Vertreter: Alge, Daniel, Mag. Dr. rer.nat.
(86) Internationale Anmeldenummer: PCT/AT1999/000271
(87) Internationale Veröffentlichungsnummer: WO 2000/027425

(56) Entgegenhaltungen:
- EP-A- 0 519 900
- WO-A-94/14471
- WO-A-95/25536
- WO-A-97/34930
- WO-A-98/32459
- NEELS ET AL: "ligand receptor interaction of the low density lipoprotein receptor-related protein, a multi-ligand endocytic receptor" FIBRINOLYSIS & PROTEOLYSIS, Bd. 12, Nr. 4, 1998, Seiten 219-240, XP000907114
- NOORMAN F ET AL: "regulation of tissue-type plasminogen activator concentration by clearance via the mannose receptor and other receptors" FIBRINOLYSIS & PROTEOLYSIS, Bd. 11, Nr. 4, 1997, Seiten 173-186, XP000907181
- YAKYAEV ET ALL: "cellular uptake and degradation of the thrombin activated factor VIII fragmets" BLOOD, Bd. 90, Nr. 10, suppl 1, - 1997 Seite 31a XP000907187 in der Anmeldung erwähnt
- NARITA MASAAKI ET AL: "The low-density lipoprotein receptor-related protein (LRP) mediates clearance of coagulation factor Xa in vivo." BLOOD JAN. 15, 1998, Bd. 91, Nr. 2, 15. Januar 1998 (1998-01-15), Seiten 555-560, XP002137878 ISSN: 0006-4971
- KOUNNAS MARIA Z ET AL: "Cellular internalization and degradation of antithrombin III-thrombin, heparin cofactor II-thrombin, and alpha-1-antitrypsin-trypsin complexes is mediated by the low density lipoprotein receptor-related protein." JOURNAL OF BIOLOGICAL CHEMISTRY 1996, Bd. 271, Nr. 11, 1996, Seiten 6523-6529, XP002137879 ISSN: 0021-9258
- SCHWARZ, HANS PETER ET AL: "Involvement of low-density lipoprotein receptor - related protein ( LRP ) in the clearance of factor VIII in von Willebrand factor -deficient mice" BLOOD (2000), 95(5), 1703-1708 , XP002137880

## Beschreibung

Die Erfindung betrifft eine pharmazeutische Präparation auf Basis von Blutgerinnungsproteinen und Maßnahmen zur Verlängerung von deren biologischer Halbwertszeit.

Therapeutische Proteine, insbesondere Präparationen zur Behandlung von Blutgerinnungsstörungen, welche Blutgerinnungsproteine, wie z.B. von Willebrand-Faktor (vWF), beinhalten, haben oft relativ kurze Halbwertszeiten im Organismus. Dadurch kann eine therapeutische Verabreichung solcher Präparationen innerhalb kurzer Zeit wirkungslos werden oder zumindest in ihrer Wirksamkeit stark beeinträchtigt werden.

Vieles an den physiologischen Abbauwegen von Blutgerinnungsproteinen ist ebenso unbekannt wie die genauen Faktoren, die deren Halbwertszeit im Körper bestimmen.

So ist lediglich bekannt, daß aktivierter Faktor VIII über seine A2-Domäne an das Lipoproteinrezeptor-verwandte Protein (lipoprotein receptor related protein; LRP) bindet, wobei diese Bindung sowohl für die Internalisierung von Faktor VIII als auch für dessen Abbau zuständig ist. Es ist auch bekannt, daß durch ein Rezeptor-assoziiertes Protein (RAP), einem Inhibitor von LRP, die Internalisierung der A2-Domäne von Faktor VIII gehemmt werden kann, wobei angenommen wird, daß die Dissoziation der A2-Untereinheit vom übrigen aktivierten Faktor VIII-Molekül reversibel ist.

Das "low density lipoprotein related protein" ist ein multifunktionell endozytotisch aktiver Rezeptor, der strukturell wie funktionell unterschiedliche Liganden bindet und endozytieren kann. RAP ("receptor-associated protein") inhibiert alle Interaktionen der Liganden mit LRP in vitro.

Prinzipiell wird vermutet, daß dieser Mechanismus von Bedeutung bei der Aktivitätsregulierung von Faktor VIIIa sein könnte (Blood 90 (10) Suppl. 1: 31a (1997)).

Es ist auch bekannt, daß LRP als Abbaurezeptor (Clearence receptor) für Gewebeplasminogenaktivator (tPA) fungieren kann, d.h. für dessen Entfernung aus der Blutzirkulation verantwortlich ist. Weiters scheinen dabei auch weitere Rezeptoren, wie z.B. der Mannose-Rezeptor, von Bedeutung zu sein. Andererseits ist auch bekannt, daß LRP bei der Clearence von vielen verschiedenen Liganden, wie Proteinasen, Inhibitoren, deren Komplexe mit Proteinasen, sowie verschiedenen Lipoproteinen, beteiligt sein kann. LRP kann sowohl zellgebunden vorliegen als auch als lösliche Form (Quinn K.A. et al., XVIIth Congress of the International Society of Thrombosis and Haemostasis, 1999, Abstract).

Es wurde gefunden, daß tPA eine sehr schnelle Clearence im Blutkreislauf erfährt, und daß Mutanten von tPA sowie Inhibitoren der Clearence dazu verwendet werden können, um die Dosierung von tPA bei der Thrombolysetherapie zu verringern (Fibrinolysis and Proteolysis (1997), S. 173-186).

In RAP-defizienten Mäusen konnte nachgewiesen werden, daß die Clearence von α₂-Makroglobulin in der Leber gestört ist. Hierbei wurde auch nachgewiesen, daß die Menge von reifem prozessierten LRP sowohl in der Leber als auch im Gehirn reduziert ist (PNAS, 92 (1995), S. 4537-4541).

In der EP-0 713 881-A2 werden von Willebrand-Faktor (vWF)-Konzentrate in Kombination mit Antithrombolytika und/oder Fibrinolytika beschrieben. Zweck dieser Präparate ist die Verringerung der Blutungsrisiken bei einer Antikoagulationstherapie.

In der US 5 304 383 wird eine pharmazeutische Präparation, enthaltend lys-Plasminogen in Kombination mit einem Serinproteaseinhibitor, wie Aprotinin oder α₂-Makroglobulin, beschrieben, welche zur Behandlung von Plasminogen-Mangel und Thrombosen eingesetzt werden kann.

Die Erfindung stellt sich zur Aufgabe, eine pharmazeutische Präparation zur Verfügung zu stellen, auf Basis von einem Blutgerinnungsprotein, welches sich durch eine verbesserte in vivo-Halbwertszeit auszeichnet. Die Erfindung stellt sich weiters zur Aufgabe, die in vivo Halbwertszeit eines Blutgerinnungsproteins zu verlängern, um einerseits den endogenen Gehalt an dem Blutgerinnungsprotein zu stabilisieren und andererseits die Effizienz von einem exogenen Blutgerinnungsprotein zu steigern.

Die Aufgabe wird erfindungsgemäß durch eine pharmazeutische Präparation zur Behandlung von Blutgerinnungsstörungen gelöst, welche Präparation mindestens ein pro-Protein der Blutgerinnung, ausgesucht aus der Gruppe bestehend aus Faktor VIII, von Willebrand-Faktor (vWF) oder Faktor V und weiters einen gerinnungsphysiologisch inerten Rezeptor-Bindungskompetitor ausgewählt aus der Gruppe bestehend aus RAP, einer Mutante von RAP, einem Analogen von RAP und der Kombination aus dem Tissue-Typ Plasminogenaktivator (tPA) und Aprotinin. Dadurch wird die Wirkung des Proteins auf die Blutgerinnung nicht unmittelbar beeinflußt, der Rezeptor-Bindungskompetitor trägt lediglich zur Stabilisierung des Proteins in vivo bei.

Ein erfindungsgemäß vorgesehenes pro-Protein der Blutgerinnung in der erfindungsgemäßen Präparation ist vWF bzw. dessen analoge Proteine. vWF zeigt vor allem in Plättchen-reichem Plasma seine Eigenständigkeit als Blutgerinnungsfaktor, unabhängig von seiner Rolle als Träger und Stabilisator des Faktor VIII (Beguin et al., Thrombosis and Haemostasis 78 (1), 590-594 (1997)). vWF wird beispielsweise aus Plasma oder einer Plasmafraktion gewonnen, kann aber auch durch rekombinante DNA-Technologie, durch Expression einer transformierten Zelle, hergestellt werden. Beispielsweise wird ein rekombinanter vWF nach der Vorschrift von Pannekoek (EP 0 197 592) bzw. Fischer et al. (WO 96/10584) hergestellt.

Die Gruppe der Blutgerinnungszymogene bzw. Vorstufen der gerinnungsphysiologisch aktiven Blutgerinnungsproteine sind einerseits aus Plasma oder einer Plasmafraktion erhältlich und entsprechen den nativen Proteinen. Andererseits können die nativen Proteine bzw. deren Derivate auch aus Zellkulturüberständen gewonnen werden. Die Zellen werden dabei vorzugsweise durch rekombinante DNA-Technologie transformiert, um die rekombinanten Formen der Blutgerinnungsproteine zu erhalten.

Mutanten bzw. Analoge sind insoweit in der erfindungsgemäßen Präparation enthalten, wie diese noch zur Behandlung von Blutgerinnungsstörungen eingesetzt werden können und an einen Rezeptor funktionell, also kompetitiv, binden können.

Als Rezeptor für die Proteine der erfindungsgemäßen Präparation ist vor allem der Lipoproteinrezeptor zu nennen bzw. LRP. Ein weiterer Rezeptor, an dem die Proteine binden, ist beispielsweise der Mannose-Rezeptor. Es hat sich erfindungsgemäß überraschenderweise herausgestellt, daß die Liganden des LRP vor allem für pro-Proteine der Blutgerinnung eine positive Wirkung in vivo zeigen, indem diese Liganden an den Rezeptor kompetitiv binden. Dies war insofern überraschend, da nicht vermutet werden konnte, daß pro-Proteine der Blutgerinnung und vWF an LRP binden können. LRP war vor allem zur Bindung von Enzymen bzw. deren Komplexe mit Inhibitoren bekannt. Durch die kompetitive Eigenschaft der Bindung an LRP wird die vorzeitige Bindung des Proteins in der pharmazeutischen Präparation bzw. des endogenen Blutgerinnungszymogens und vWF verhindert, wodurch dessen biologische Verfügbarkeit wesentlich verbessert wird.

Als Rezeptor-Bindungskompetitor können eine Reihe von Proteinen eingesetzt werden, wobei diejenigen bevorzugt sind, deren physiologische Toleranz geprüft ist.

Der kompetitiv wirkende Ligand ist vorzugsweise ausgewählt unter den physiologischen Proteinen, die vorzugsweise in der Zirkulation als (humanphysiologische) extrazelluläre Proteine auftreten. Diese können in nativer Form eingesetzt werden, oder als deren Analoge mit der Eigenschaft der Bindung an den Rezeptor.

Bevorzugterweise handelt es sich bei den kompetitiv wirkenden Liganden um RAP oder ein Derivat davon. Besonders bevorzugt ist ein Fragment von RAP, das jene Domäne umfaßt, die die Bindung von Proteinen an LRP verhindert und dadurch die Internalisierung und Clearance der Proteine inhibiert (I. Warshawsky et al., J. Biol.Chem., 1993, vol. 15, pp 22046-22054; Bu G., EMBO J., 1995, vol. 14, pp. 2269-2280). Es kann sich dabei um RAP oder ein Derivat davon handeln, das humanen Ursprungs ist, aber auch um tierisches RAP oder ein Derivat davon, beispielsweise aus Mäusen, Ratten oder anderen Tieren.

Um die Wirkung der pharmazeutischen Präparation bzw. der endogenen Proteine nicht zu beeinträchtigen, ist der Rezeptor-Bindungskompetitor ausgewählt unter den gerinnungsphysiologisch inerten Proteinen, darunter beispielsweise "low density lipoprotein" (LDL) und Apolipoprotein.

Als gerinnungsphysiologisch inerter Rezeptor-Bindungskompetitor können auch Gemische dieser Rezeptor-Bindungskompetitoren eingesetzt werden, die eventuell nicht als einzelne Proteine, jedenfalls aber im Gemisch keinen unerwünschten Einfluß auf das Gerinnungssystem ausüben. Beispielsweise ist ein bevorzugtes Gemisch tPA und Aprotinin, welches sich hervorragend zur Stabilisierung etwa des Faktor VIII in vivo eignet. Der gewählte Rezeptor-Bindungskompetitor ist hier also ein Ligand, dessen gerinnungsphysiologische Wirkung unterbunden ist, gegebenenfalls durch die Antagonisierung mit dem entsprechenden Inhibitor.

Anstelle eines Gemisches aus einem prinzipiell zwar wirksamen Rezeptor-Bindungskompetitor und einem geeigneten Antagonisten kann auch eine mutierte Form des Rezeptor-Bindungskompetitors verwendet werden, der durch geeignete Mutationen (z.B. im aktiven Zentrum) inaktiviert ist (dessen Affinität gegenüber dem Rezeptor aber nicht wesentlich verändert (verringert) ist.) Beispiele hierfür sind u.a. mutierte Formen von tPA, Urokinase, Kallikrein, Renin, Thrombin (WO 96/41868) etc.

Es war überraschend, daß rekombinanter tPA (rtPA) den endogenen Faktor VIII-Gehalt in einem vWF-defizienten Hund über einen längeren zeitraum stabilisierte, wodurch die Hämophilie behandelt werden konnte. Obwohl der natürliche Stabilisator des Faktor VIII, nämlich vWF, fehlte, konnte der LRP-Ligand bzw. das Gemisch der LRP-Liganden die Funktion des vWF übernehmen. Der Faktor VIII-Gehalt wurde um ca. 50 % erhöht und blieb bei diesem Niveau mehrere Tage. Das Enzym tPA ist ein bekannter Ligand des LRP. Die Verabreichung des rtPA an ein Säugetier blockiert also LRP und verhindert somit den metabolischen Abbau der Gerinnungsfaktoren, wie z.B. Faktor VIII. Dieser blockierende Effekt wird noch durch Aprotinin verstärkt. Der Effekt von Aprotinin alleine konnte auch in einem vWF-defizienten Hund gezeigt werden, der eine Kombination von rvWF und Aprotinin erhielt. Dadurch wurde wiederum eine Erhöhung des Faktor VIII-Gehaltes über einen längeren Zeitraum bewirkt.

In vielen Fällen wird ein Patient mit einem Mangel an einem bestimmten Protein dann mit dem jeweiligen Rezeptor-Bindungskompetitor behandelt, um die Bildung des endogenen Proteins zu ermöglichen bzw. das Protein zu stabilisieren. Dies ist bei Patienten mit einem phänotypischen Gerinnungsproteinmangel möglich.

Das erfindungsgemäße Präparat bzw. die genannten Wirksubstanzen werden vorzugsweise als behandelte Präparate bzw. Proteine eingesetzt, um eine Übertragung von möglicherweise vorhandenen Pathogenen, wie Viren oder Prionen (TBE), auszuschließen. Bei der Gewinnung aus biologischen Materialien, wie Blut, Plasma, Plasmafraktionen oder Zellkulturen, existiert ein Risiko der Kontamination mit Humanpathogenen, das jedoch durch eine entsprechende Behandlung zur Inaktivierung bzw. Abreicherung eliminiert werden kann. Zu den effektiven Maßnahmen zur Inaktivierung von Viren zählen beispielsweise die Behandlung mit organischen Lösungsmitteln und/oder Detergenzien (EP-0 131 740, EP-0 050 061, virus inactivating eluent), die Behandlung mit chaotropen Mitteln (WO 90 15 613) Hitzebehandlungsverfahren, vorzugsweise in lyophilisiertem, trockenem oder feuchtem Zustand (siehe EP-0 159 311), Kombinationsverfahren wie das der (EP-0 519901) und physikalische Methoden. Letztere bewirken die Inaktivierung von Viren, beispielsweise durch Bestrahlung mit Licht, etwa in Gegenwart von Photosensibilisatoren (EP-0 471 794 und WO/AT 97/00068).

Zu dem Abreicherungsverfahren der Humanpathogene zählen insbesondere die Filtrationen unter Verwendung von Ultrafiltern, Tiefenfiltern oder Nanofiltern (A 341/98). Aber auch Fällungsschritte bzw. andere Proteinreinigungsmaßnahmen, wie die der Adsorption, tragen zur Abreicherung von möglicherweise vorhandenen Pathogenen bei.

Die erfindungsgemäße Präparation kann einerseits als fertige Formulierung, d.h. als Gemisch des Proteins mit dem Rezeptor-Bindungskompetitor zur Verfügung gestellt werden. Andererseits ist es auch möglich, ein Set zur Verfügung zu stellen, welches A) das Protein und B) den Rezeptor-Bindungskompetitor enthält. Das Set hat den Vorteil, daß die Dosis der einzelnen Komponenten variabel ist und die Form der Administration den vorliegenden Verhältnissen angepaßt werden kann. Beispielsweise ist neben der iv-Verabreichung auch die intramuskuläre, subkutane oder orale Verabreichung der Wirkstoffe möglich. Die wirksubstanzen können gemeinsam, also simultan, aber auch parallel oder konsekutiv dem Patienten verabreicht werden. Bei Verwendung eines Sets ist die erfindungsgemäße Präparation vorzugsweise in Fertigspritzen mit den einzelnen Komponenten vorgefertigt.

Eine erfindungsgemäße Indikation für das Präparat ist beispielsweise eine Behandlung eines Patienten mit einem phänotypischen Gerinnungsfaktormangel, z.B. eines vWF-defizienten Patienten. Dabei wird erfindungsgemäß ein Präparat hergestellt auf Basis des Gerinnungsfaktors, der im Patienten mangelhaft ist, mit der erfindungsgemäßen Kombination des Rezeptor-Bindungskompetitors. Aber auch der Rezeptor-Bindlungskompetitor alleine entspricht der erfindungsgemäßen Indikation. Beispielsweise wird bei einem Mangel an funktionellem Faktor VIII der Rezeptor-Bindungskompetitor für Faktor VIII verabreicht, welcher eventuell das Gemisch tPA und Aprotinin ist.

Gegenstand der vorliegenden Erfindung ist auch die Verwendung einer LRP-Ligandenpräparation zur Herstellung eines Mittels zur Behandlung von phänotypischem Gerinnungsfaktormangel (z.B. vWF-, Faktor VIII- oder Faktor IX-Mangel) oder zur Verlängerung der biologischen Halbwertszeit eines pro-Proteins der Blutgerinnung, ausgewählt aus der Gruppe bestehend aus Faktor VIII, vWF und Faktor V.

Die Erfindung wird an Hand der nachfolgenden Beispiele und der Zeichnungen, auf die sie jedoch nicht beschränkt sein soll, näher erläutert.

Es zeigen:
- Fig. 1:: Faktor VIII-Bestimmung im vWF-defizienten Hund
- Fig. 2:: vWF und Faktor VIII-Bestimmung in vWF-defizientem Hund

### Beispiele:

### Beispiel 1 :

Ein Kooiker Hund mit schwerem von Willebrand Faktor-Mangel vom Typ 3 (Rieger et al., Thromb. Haemost. (1998), 80: 332-337) männlichen Geschlechts, 8,5 kg Körpergewicht, 2 Jahre alt, wurde mit 10 mg/kg Körpergewicht Ketamin (Ketasol, Dr. E. Gräub AG, Bern, Schweiz) und 1 mg/kg Xylaxin (Xylasol, Dr. E. Gräub AG, Bern, Schweiz) narkotisiert. Anschließend wurde ein permanenter venöser Zugang über einen Unterarm geschaffen und über eine Kanüle Aprotinin (Pantinol 100.000 K.I.E. Ampullen, Gerot Pharmazeutika, Wien) in einer Dosis von 10 000 K.I.E. pro kg Körpergewicht als Bolus verabreicht. Anschließend wurde rekombinanter Gewebeplasminogenaktivator (Actilyse, Boehringer Mannheim) (rtPA) gemäß Herstellerangabe mit destilliertem Wasser rekonstituiert und dem von Willebrand-defizienten Hund in einer Dosis von 0,25 mg/kg Körpergewicht als Bolus verabreicht. Gleichzeitig wurde eine Blutprobe auf Zitrat entnommen. Innerhalb der ersten Stunde der Behandlung wurde eine Coinfusion von Aprotinin (3000 K.I.E. pro kg Körpergewicht) und rtPA 0,75 mg/kg Körpergewicht verabreicht und nach einer Stunde wurde auf eine Infusion gewechselt, die Aprotinin in einer Dosis von 3000 K.I.E. pro kg Körpergewicht und rtPA von 0,5 mg/kg Körpergewicht enthielt, und diese über eine Stunde verabreicht. Anschließend, d.h. nach zwei Stunden, wurde über eine weitere Stunde Aprotinin in einer Dosis von 3000 K.I.E./kg Körpergewicht infundiert. Zu den Zeitpunkten 30 min, 1, 3, 24, 48, 92 und 96 Stunden nach Versuchsbeginn wurden Blutproben auf Zitrat abgenommen und durch Zentrifugation daraus plättchenarmes Plasma hergestellt und bei -20°C tiefgefroren und bis zur weiteren Analyse gelagert.

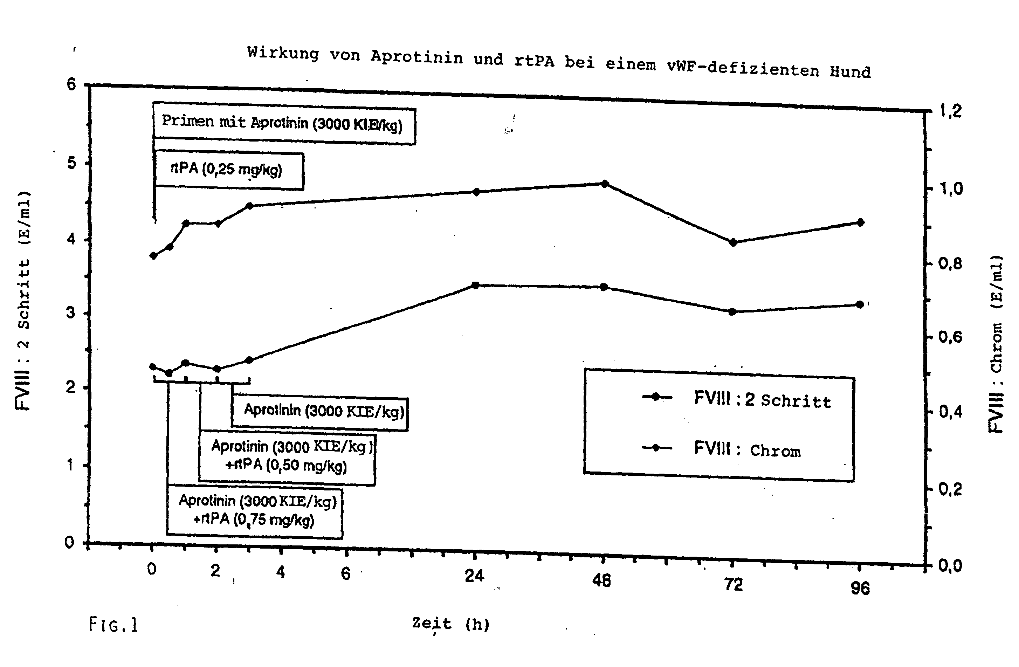

In den gefrorenen Blutproben wurde nach Abschluß des Infusionsexperimentes Blutgerinnungsfaktor VIII mit zwei unterschiedlichen Bestimmungsmethoden quantitativ bestimmt. Erstens wurde die Zweistufengerinnungsmethode gemäß der Methode von Austen, D.E.G. und Rhymes, I.L., A Laboratory Manual of Blood Coagulation, Oxford, UK, Blackwell Scientific, (1975), unter Verwendung der Reagentien des Zweistufen-Faktor VIII - Test-Kits der IMMUNO AG, Wien, verwendet. Zweitens wurde Faktor VIII mit dem chromogenen Faktor VIII-Test-Kit, Immunochrom FVIII:C der Immuno AG, Wien, bestimmt (Lang H., Oberreiter M., Moritz, B. Thromb. Haemost. 65:943, (1991)). Faktor VIII wurde gegen einen humanen Plasma Faktor VIII-Standard, der gegen den dritten internationalen Standard 91/666 kalibriert worden war, gemessen. Die Faktor VIII-Aktivität wird in humanen Faktor VIII-Einheiten pro ml ausgedrückt. Das Resultat dieser Bestimmung ist in der Fig. 1 zusammengefaßt. In den beiden unabhängig voneinander durchgeführten Faktor VIII-Bestimmungsmethoden zeigte sich ein Anstieg der Faktor VIII-Plasmaaktivität, der über einen Zeitraum von 24 bis 48 Stunden anhielt und anschließend auf einem Niveau von ca. 150 % des Ausgangswertes, unabhängig von der verwendeten Testmethode, über einen Zeitraum bis zu 96 Stunden nach Versuchsbeginn konstant blieb. Da bekannt ist, daß von Willebrand-Faktor gegen den proteolytischen Abbau durch Plasmin empfindlich ist, mußte angenommen werden, daß die Gabe von rtPA eine vermehrte von Willebrand-Faktor-Inaktivierung, verbunden mit einer sekundären Reduktion des Plasma-Faktor VIII-Spiegels durch Fehlen des Stabilisierungsproteins für Faktor VIII zu finden sein wird. Die gleichzeitige Gabe von Aprotinin als Inhibitor der Fibrinolyse und des Plasmins ließ lediglich eine Inhibition des rtPA-Effektes erwarten. Der scheinbar paradoxe Anstieg des Plasma-Faktor VIII-Levels kann daher durch die Interferenz von Aprotinin und/oder rtPA mit den Metabolisierungsmechanismen für Faktor VIII erklärt werden.

### Beispiel 2 :

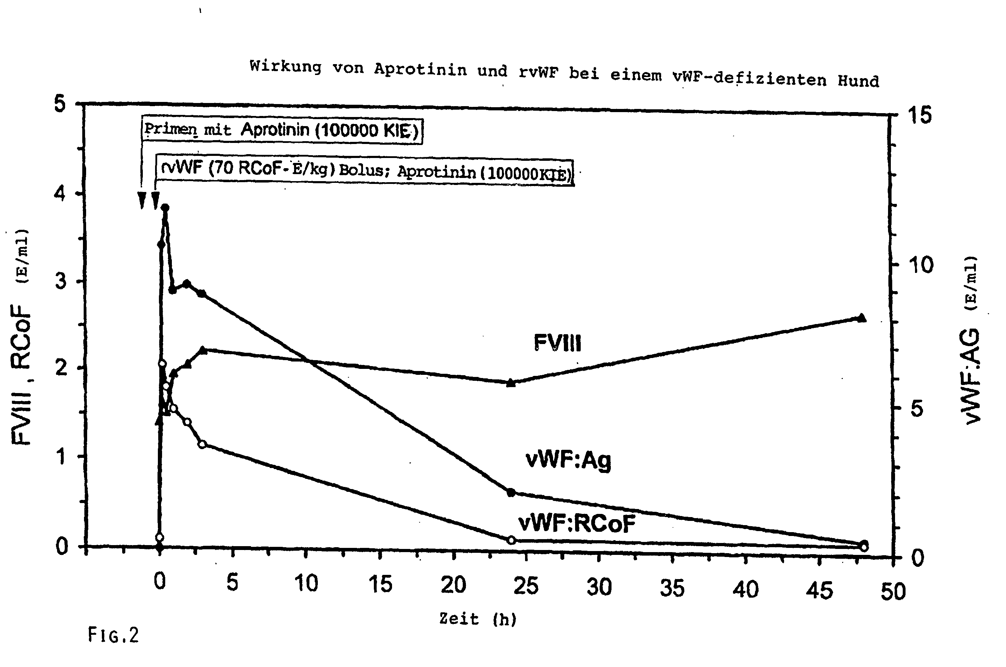

Ein von von Willebrand-Faktor-defizienter Hund wie in Beispiel 1 wurde narkotisiert und anschließend mit Aprotinin (Pantinol) mit einer Dosis von 100.000 K.I.E. als intravenöser Bolus vorbehandelt. Anschließend wurde dem Tier ein rekombinantes von Willebrand-Faktor-Präparat in einer Dosis von 70 RcoF E/kg gegeben. Die Herstellung und Charakterisierung des rekombinanten von Willebrand-Faktor-Präparates ist in Fischer et al., FEBS Lett. 375:259 (1995), beschrieben. Unmittelbar nach der Gabe des rekombinanten von Willebrand-Faktor-Präparates wurde über drei Stunden eine Infusion von 100.000 K.I.E. Aprotinin intravenös verabreicht. Blutproben auf Zitrat wurden vor Beginn des Versuches (= Zeitpunkt 0) 15 min, 30 min, 1 h, 3 h, 24 h und 48 h nach der Behandlung mit rekombinantem von Willebrand-Faktor entnommen und daraus Plasma, wie in Beispiel 1 beschrieben, hergestellt und bis zur weiteren Analyse bei -20°C tiefgefroren. Von diesen Blutproben wurde anschließend von Willebrand-Faktor-Antigen unter Verwendung eines Kaninchen Anti-Human-von Willebrand-Faktor-Antikörpers mit dem Test Asserachrom vWF, Boehringer Mannheim, gemessen. Außerdem wurde die Ristocetin-Cofaktor-Aktivität, gemessen durch Ristocetin induzierte Aggregation Formaldehyd-fixierter humaner Blutplättchen und nach der Methode von Evans und Austen, Brit. J. Hetamol. 37:289 (1977) bestimmt. Als von Willebrand-Faktor-Standard diente ein humaner Plasma-Standard. Außerdem wurde Faktor VIII, wie in Beispiel 1 beschrieben, getestet. Die Resultate der Bestimmung der Blutproben an von Willebrand-Faktor und Faktor VIII-Aktivität sind der Fig. 2 zu entnehmen. In der Grafik ist ein Mittelwert der Faktor VIII-Aktivität, gemessen nach den beiden Faktor VIII-Bestimmungsmethoden, als Graph eingezeichnet. Von Willebrand-Faktor wurde, wie aus der Literatur bekannt (Turecek et al., Blood 90: 3555 (1997)), mit einer Halbwertszeits von 10 bis 20 h eliminiert, so daß nach 48 h kaum mehr von Willebrand-Faktor in der Zirkulation nachweisbar war. Gleichzeitig mit Gabe des rekombinanten von Willebrand-Faktors und des Aprotinins stieg der Plasma-Faktor VIII-Spiegel innerhalb von 3 h auf ca. 150 % des Ausgangswertes an und blieb dann auf diesem Niveau stabil oder zeigte sogar einen leicht steigenden Trend bis zu 48 h nach der Verabreichung. Auch dieses Ergebnis kann wie Beispiel 1 interpretiert werden, daß durch die Gabe des Aprotinins trotz fehlender Stabilisierung des Faktor VIII durch den rekombinanten von Willebrand-Faktor, durch eine Interferenz mit dem Faktor VIII-Metabolismus der Faktor VIII-Plasma-Spiegel anhaltend erhöht bleibt.

### Beispiel 3 :

### Wirkung von RAP auf Faktor VIII-Gewinnung in Knock-out-Mäusen mit schwerem Faktor VIII-Mangel

Auf gentechnischem Wege wurde ein Mäusestamm mit schwerem Faktor VIII (FVIII)-Mangel durch gezielte Disruption des Mäuse-Faktor VIII-Gens gemäß Bi et al. Nature Genetics 10:119-121 (1995) geschaffen. Faktor VIII-Knock-out-Mäuse wurden durch eine Insertion eines neo-Gens in das 3'-Ende von Exon 17 des Mäuse-Faktor VIII-Gens geschaffen. Die betroffenen männlichen Tiere (X'Y) hatten nicht nachweisbare Faktor VIII-Levels von <0,02 ± 0,01 E/ml, wenn Messungen entweder mit einem chromogenen Faktor VIII-Test, Hyland Immuno, Wien, Österreich, wie kürzlich beschrieben (Turecek et al., Thromb. Haemostas. Suppl. 769 (1997)) oder mittels Antigen-ELISA, wie nachstehend beschrieben, vorgenommen wurden.

Zwei betroffene, hemizygote männliche Mäuse (X'Y) wurden intravenös mit einer Dosis von 200 E/kg Körpergewicht einer rekombinanten Human-Faktor VIII (rhFVIII)-Präparation behandelt, die von Chinese Hamster-Ovarien-Zellen stammte, die wie beschrieben hergestellt worden waren (PCT WO/85/01961), und ohne stabilisierendes Protein pharmazeutisch formuliert worden war.

Unter Narkose wurden eine Stunde nach der Behandlung die Schwanzspitzen der Mäuse mit der Klinge eines Skalpells, wie von Novak et al., Brit. J. Haematol. 69:371-378 (1998) beschrieben, eingeschnitten. Ein Volumen von 50 µl Blut wurde mit Kapillarröhrchen (Ringcaps, Hirschmann, Deutschland) aus den Schwanzwunden gesammelt, wobei die Kapillarröhrchen mit Lithium-Heparin als Antikoagulans beschichtet waren. Die Kapillarröhrchen wurden geschlossen und zentrifugiert, um die Blutzellen vom Plasma zu trennen. Danach wurden die Kapillarröhrchen geöffnet, und die Zell- und die Plasma-Fraktion wurden durch weiteres Zentrifugieren gesammelt. Schließlich wurden die Plasmaproben der Faktor VIII-Bestimmung mittels Faktor VIII-Antigen ELISA, Test-Set IMMUNOZYM FVIII Ag, Hyland Immuno, Wien, Österreich, unter Verwendung von monoklonalen Antifaktor VIII-Antikörpern sowohl für das Abfangen wie für den Nachweis, wie beschrieben (Stel et al., Nature 303:530-532 (1983); Lenting et al., J. Biol. Chem. 269: 7150-7155 (1994); Leyte et al., Biochem. J. 263:187-194 (1989)) unterzogen. Die resultierenden Faktor VIII-Werte wurden in internationalen Einheiten von humanem Faktor VIII ausgedrückt. Die Ergebnisse der Faktor VIII-Plasma-Level sind in der Tabelle angegeben.

Zwei andere betroffene hemizygote männliche Mäuse (X'Y) wurden mit rekombinantem Rezeptor-assoziiertem Protein (GST-RAP) 10 min vor der Behandlung mit dem rekombinanten humanen Faktor VIII mit einer Dosis von 40 mg/kg Körpergewicht vorbehandelt. Das bei dieser Untersuchung verwendete Rezeptor-verwandte Protein (RAP), welches mit dem Low-Density-Lipoprotein-Rezeptor in Wechselwirkung tritt, wurde durch bakterielle Fermentation, wie von Hertz et al., J. Biol. Chem. 266:21232-21238 (1991) beschrieben, erhalten. Ein Fusionsprotein des RAP mit Glutathion-S-Transferase wurde in E. coli exprimiert und mittels Affinitätschromatographie auf Glutathion-Agarose gereinigt. Das resultierende Protein bestand hauptsächlich aus dem Fusionsprotein und Spaltprodukten von RAP und Glutathion-S-Transferase. Das Fusionsprotein wurde in einem injizierbaren Puffer bereit für eine Verabreichung an die Faktor VIII-Knock-out-Mäuse formuliert. Wie bei der Kontrollgruppe (Behandlung nur mit Faktor VIII) wurden Blutproben eine Stunde nach der Verabreichung von rekombinantem Faktor VIII genommen und unter Verwendung von Faktor VIII-Antigen-ELISA auf Faktor VIII-Aktivität gemessen. Die Ergebnisse sind in der Tabelle angeführt.

| | Behandlung | Dosis | Gewinnung 1 h nach Behandlunq |
|---|---|---|---|
| Maus Nr. | GST-RAP | rhFVIII | FVIII:Ag (E/ml Plasma) |
| 1 | 40 mg/kg | 200 E/kg | 1,92 |
| 2 | 40 mg/kg | 200 E/kg | 1,88 |
| 3 | - | 200 E/kg | 0,73 |
| 4 | - | 200 E/kg | 0,83 |

Bei mit GST-RAP vorbehandelten Mäusen betrug die Faktor VIII-Gewinnung mehr als 200 % der Plasma-Level nach der Behandlung mit rekombinantem Faktor VIII allein.

## Patentansprüche

1. Pharmazeutisches Präparat zur Behandlung von Blutgerinnungsstörungen, enthaltend mindestens ein pro-Protein der Blutgerinnung; ausgesucht aus der Gruppe bestehend aus Faktor VIII, von Willebrand Faktor (vWF) oder Faktor V und weiters einen gerinnungsphysiologisch inerten Rezeptor-Bindungskompetitor ausgewählt aus der Gruppe bestehend aus RAP, einer Mutante von RAP, einem Analogen von RAP und der Kombination aus dem Tissue-Typ Plasminogenaktivator (tPa) und Aprotinin.

2. Präparat nach Anspruch 1, **dadurch gekennzeichnet, dass** das pro-Protein aus einem biologischen Material ausgesucht aus der Gruppe von Humanplasma, einer Plasmafraktion und einem Zellkulturüberstand gewonnen ist.

3. Präparat nach Anspruch 1 oder 2, **dadurch gekennzeichnet, dass** es als Set zur Verfügung gestellt wird, enthaltend
a) das pro-Protein der Blutgerinnung und
b) den Rezeptor-Bindungskompetitor.

4. Präparat nach einem der Ansprüche 1 bis 3, **dadurch gekennzeichnet, dass** das pro-Protein der Blutgerinnung Faktor VIII ist und der Rezeptor-Bindungskompetitor ein Gemisch aus Aprotinin und tPA ist.

5. Präparat nach einem der Ansprüche 1 bis 3, **dadurch gekennzeichnet, dass** das pro-Protein der Blutgerinnung vWF ist und der Rezeptor-Bindungskompetitor ein Gemisch aus Aprotinin und tPA ist.

6. Kombinationspräparat enthaltend Aprotinin und tPA zur medizinischen Verwendung.

7. Verwendung eines Präparats nach einem der Ansprüche 1 bis 5 zur Herstellung eines Mittels zur Behandlung eines Patienten mit einem phänotypischen Gerinnungsfaktor-Mangel.

8. Verwendung nach Anspruch 7, **dadurch gekennzeichnet, dass** der Patient vWF-defizient ist.

9. Verwendung eines Präparates nach einem der Ansprüche 1 bis 5 zur Herstellung eines Mittels zur Verlängerung der biologischen Halbwertszeit eines pro-Proteins der Blutgerinnung, ausgesucht aus der Gruppe bestehend aus Faktor VIII, vWF und Faktor V in vivo.

10. Verwendung eines Präparats nach Anspruch 9 enthaltend Aprotinin und tPA.

11. Verwendung nach Anspruch 9, **dadurch gekennzeichnet, dass** das pro-Protein Blutgerinnungsfaktor VIII ist.

12. Verwendung eines pharmazeutischen Präparates enthaltend einen gerinnungsphysiologisch inerten Rezeptor-Bindungskompetitor ausgewählt aus der Gruppe bestehend aus RAP, einer Mutante von RAP, einem Analogen von RAP und der Kombination aus dem Tissue-Typ Plasminogenaktivator (tPa) und Aprotinin, zur Herstellung eines Mittels zum Verlängern der biologischen Halbwertszeit eines Proteins ausgewählt aus der Gruppe bestehend aus Faktor VIII, Faktor V und vWF.

## Claims

1. A pharmaceutical preparation for the treatment of blood coagulation disorders containing at least one pro-protein of blood coagulation, selected from the group consisting of factor VIII, von Willebrand factor (vWF) or factor V, and, in addition, a coagulation physiologically inert receptor binding competitor selected from the group consisting of RAP, a RAP mutant, a RAP analogue and the combination of tissue type plasminogen activator (tPA) and aprotinin.

2. A preparation according to claim 1, **characterized in that** said pro-protein is derived from a biological material selected from the group of human plasma, a plasma fraction and a cell culture supernatant.

3. A preparation according to claim 1 or 2, **characterized in that** it is provided as a set containing
a) said pro-protein of blood coagulation and
b) said receptor binding competitor.

4. A preparation according to any one of claims 1 to 3, **characterized in that** said pro-protein of blood coagulation is factor VIII and said receptor binding competitor is a mixture of aprotinin and tPA.

5. A preparation according to any one of claims 1 to 3, **characterized in that** said pro-protein of blood coagulation is vWF and said receptor binding competitor is a mixture of aprotinin and tPA.

6. A combination preparation containing aprotinin and tPA for medical use.

7. The use of a preparation according to any one of claims 1 to 5 for the preparation of an agent to treat a patient suffering from phenotypic coagulation factor deficiency.

8. The use according to claim 7, **characterized in that** said patient is vWF deficient.

9. The use of a preparation according to any one of claims 1 to 5 for the preparation of an agent to extend the biological *in vivo* half-life of a pro-protein of blood coagulation selected from the group consisting of factor VIII, vWF and factor V.

10. The use of a preparation according to claim 9 containing aprotinin and tPA.

11. The use according to claim 9, **characterized in that** said pro-protein is blood coagulation factor VIII.

12. The use of a pharmaceutical preparation containing a coagulation physiologically inert receptor binding competitor selected from the group consisting of RAP, a RAP mutant, a RAP analogue and the combination of tissue type plasminogen activator (tPA) and aprotinin for producing an agent to extend the biological half-life of a protein selected from the group consisting of factor VIII, factor V and vWF.

## Revendications

1. Préparation pharmaceutique pour le traitement de troubles de la coagulation sanguine contenant au moins une pro-protéine de la coagulation sanguine choisie dans le groupe constitué par le facteur VIII, le facteur Willebrand (vWF) ou le facteur V, et, de plus, un antagoniste de récepteur physiologiquement inerte à la coagulation choisi dans le groupe constitué par une protéine RAP, un mutant de la protéine RAP, un analogue de la protéine RAP et la combinaison de l'activateur tissulaire du plasminogène (tPA) et de l'aprotinine.

2. Préparation selon la revendication 1, **caractérisée en ce que** la pro-protéine est extraite d'un matériau biologique choisi dans le groupe comprenant le plasma humain, une fraction de plasma et un surnageant de culture de cellules.

3. Préparation selon les revendications 1 ou 2, **caractérisée en ce qu'**elle est mise à disposition comme un set contenant
a) la pro-protéine de la coagulation sanguine, et
b) l'antagoniste de récepteur.

4. Préparation selon l'une quelconque des revendications 1 à 3, **caractérisée en ce que** la pro-protéine de la coagulation sanguine est le facteur VIII, et que l'antagoniste de récepteur est un mélange d'aprotinine et d'activateur tissulaire du plasminogène tPA.

5. Préparation selon l'une quelconque des revendications 1 à 3, **caractérisée en ce que** la pro-protéine de la coagulation sanguine est le vWF et que l'antagoniste de récepteur est un mélange d'aprotinine et de tPA.

6. Préparation de combinaison contenant l'aprotinine et le tPA pour une application médicale.

7. Utilisation d'une préparation selon l'une quelconque des revendications 1 à 5 pour la fabrication d'un agent pour le traitement d'un patient avec un manque phénotypique en facteur de coagulation.

8. Utilisation selon la revendication 7, **caractérisée en ce que** le patient est déficient en vWF.

9. Utilisation d'une préparation selon l'une quelconque des revendications 1 à 5 pour la fabrication d'un agent d'allongement de la demi-vie biologique d'une pro-protéine de la coagulation sanguine choisie dans le groupe constitué par le facteur VIII, le vWF et le facteur V in vivo.

10. Utilisation d'une préparation selon la revendication 9 contenant de l'aprotinine et du tPA.

11. Utilisation selon la revendication 9, **caractérisée en ce que** la pro-protéine est le facteur VIII de coagulation sanguine.

12. Utilisation d'une préparation pharmaceutique contenant un antagoniste de récepteur physiologiquement inerte à la coagulation choisi dans le groupe constitué par une protéine RAP, un mutant de la protéine RAP, un analogue de la protéine RAP et la combinaison de l'activateur tissulaire du plasminogène (tPA) et de l'aprotinine, pour la fabrication d'un agent d'allongement de la demi-vie biologique d'une protéine choisie dans le groupe constitué par le facteur VIII, le facteur V et le vWF.
